# EUROPEAN PATENT APPLICATION

(11) **EP 0 691 196 A1**
(43) Date of publication of application: **10.01.1996**
(21) Application number: 95109728.6
(22) Date of filing: 22.06.1995
(51) Int. Cl.: B32B 1/08, A61L 29/00, F16L 11/04

(54) **Infusion tube made of flexible material for medical applications**

(30) Priority: 07.07.1994 IT BO940318
(71) Applicant: MECANO ENGINEERING S.r.l., I-40038 Vergato (Bologna) (IT)
(72) Inventor: Rondelli, Raffaela, I-41055 Montalto Di Montese (Modena) (IT)
(74) Representative: Modiano, Guido, Dr.-Ing.

(57) **Abstract**

An infusion tube made of flexible material for medical applications, including an inner layer (2) of polypropylene externally covered with a concentric layer (3) of a material having flexibility properties and adhesion compatibility with the inner layer of polypropylene.

## Description

The present invention relates to an infusion tube made of flexible material for medical applications, for example for infusing liquids such as blood plasma and the like.

Infusion tubes for the above mentioned use are already widespread in the medical field. They are usually made of PVC (polyvinyl chloride), the elasticity characteristics whereof are sufficient to allow the application of throttling devices capable of adjusting the flow of liquid by varying the cross-section of the tube, and to allow the connection of needles, plasma bags, cannulae, and the like.

The tubes must furthermore have particular mechanical requirements to avoid the formation of elbow-shaped crimps when the radius of curvature drops below a limit value.

However, researches have shown that PVC tends to release toxic substances into the liquid that comes into contact therewith; despite being harmless for the human body in the infinitesimal doses in which they are released, there is a tendency to eliminate these substances to avoid accumulation effects in the body.

In theory, it could be conceivable to use tubes made of fully non-toxic materials, but unfortunately tubes made of these materials do not have the characteristics required to allow their application, or have excessively high material and/or transformation costs.

A principal aim of the present invention is therefore to provide a tube that is adapted to obviate the shortcomings of known tubes, that is to say, is non-toxic and flexible enough according to the requirements of use in the medical field.

Within the scope of this aim, an object of the present invention is to provide a flexible tube that can be manufactured with easily available materials having adequate costs and with conventional methods, so as to combine high performance linked to the specific use with economical advantages for the users.

Another object of the present invention is to provide a tube adapted to withstand bacteriological sterilization processes without being damaged.

This aim and these objects are achieved by an infusion tube made of flexible material for medical applications, characterized in that it comprises an inner layer of polypropylene externally covered with a concentric layer of a material having flexibility properties and adhesion compatibility with said inner layer of polypropylene.

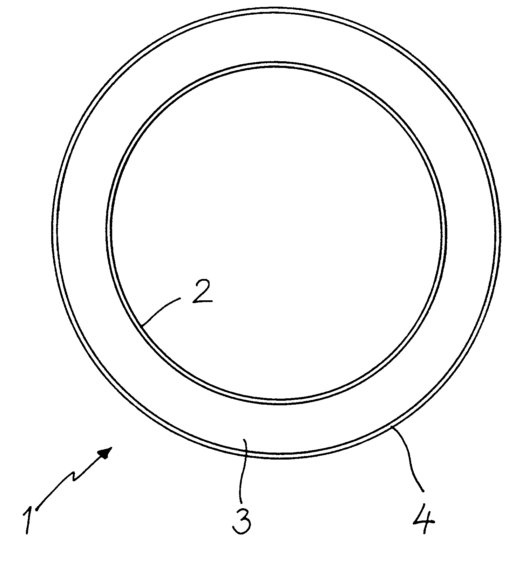

Further characteristics and advantages of the invention will become apparent from the accompanying drawing, which shows, by way of non-limitative example, a sectional view of a tube according to the present invention.

With reference to the above figure, the tube is generally designated by the reference numeral 1 and is composed of a thin inner tubular layer 2 made of a plastic material having high-level non-toxicity characteristics, for example a polypropylene.

The layer 2 internally lines an intermediate concentric layer 3 made of a plastic material having flexibility characteristics and such a thickness so as to give strength to the tube 1.

The concentric layer 3 can be externally covered with an additional protective layer 4.

In a preferred embodiment, the concentric layer 3 is constituted by a thermoplastic compound based on styrene-ethylene-butylene-styrene saturated block polymers, also known under the acronym S-EB-S.

This S-EB-S material is highly adhesion-compatible with the propylene of the inner layer 2.

Conveniently, for the same purpose of ensuring close coupling to the intermediate concentric layer 3, the outer layer 4 is also made of polypropylene.

In a preferred embodiment of the invention, with a tube having an outside diameter of 4 mm, the inner layer 2 and the outer layer 4 both have a thickness to the order of 0.1 mm and the intermediate concentric layer 3 has a thickness of approximately 0.5 mm. These values can of course vary in compliance with the required flexibility and flow-rate characteristics of the tube.

A thermoplastic elastomeric compound, commercially known under the name EVOPRENE G in the FDA version and manufactured by the British company EVODE PLASTIC LTD, in LEICESTER, UNITED KINGDOM, has turned out to be a material particularly adapted for performing the function of the intermediate layer 3.

In the practical embodiment of the invention, the tube can be manufactured by simultaneous coaxial extrusion of the three layers 2, 3, and 4, the contact surfaces whereof perfectly adhere to each other at the extrusion temperatures.

It is of course also possible to produce the tube 1 by applying the inner and outer layers 2,4 to the intermediate concentric layer 3 in subsequent steps.

In this case, adhesion of the layers between each other can be achieved with heat treatments and/or with the interposition of adapted adhesive products capable of maintaining the flexibility of the tube 1 in the course of time.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. Infusion tube made of flexible material for medical applications, characterized in that it comprises an inner layer (2) of polypropylene externally covered with a concentric layer (3) of a material having flexibility properties and adhesion compatibility with said inner layer of polypropylene.

2. Infusion tube according to claim 1, characterized in that said concentric layer (3) is constituted by a thermoplastic compound based on styrene-ethylene-butylene-styrene saturated block polymers, known under the acronym S-EB-S.

3. Infusion tube according to claim 2, characterized in that said concentric layer (3) is constituted by an elastomeric thermoplastic compound.

4. Infusion tube according to one of claims 2 and 3, characterized in that said concentric layer (3) of S-EB-S is externally covered with an additional layer (4) of polypropylene.

5. Infusion tube according to one of the preceding claims, characterized in that said inner and outer additional layers of polypropylene (2, 4) have a thickness to the order of 0.1 mm.

6. Infusion tube according to claim 5, characterized in that the concentric layer (3) that is comprised between said layers (2, 4) of propylene has a thickness of approximately 0.5 mm.
